# FASCICULE DE BREVET EUROPEEN

(11) **EP 3 630 050 B1**
(45) Date de publication et mention de la délivrance du brevet: **24.02.2021**
(21) Numéro de dépôt: 18735648.0
(22) Date de dépôt: 24.05.2018
(51) Int. Cl.: A61K 8/19, A61K 8/73, A61Q 15/00

(54) **COMPOSITION COSMÉTIQUE AQUEUSE À BASE DE BICARBONATE**
WÄSSRIGE KOSMETISCHE ZUSAMMENSETZUNG AUF BASIS VON BICARBONAT
BICARBONATE-BASED AQUEOUS COSMETIC COMPOSITION

(30) Priorité: 02.06.2017 FR 1754903
(43) Date de publication de la demande: 08.04.2020
(73) Titulaire: Laboratoires M & L, 04100 Manosque En Provence (FR)
(72) Inventeur: MILLET, Magali, 04190 Les Mees (FR); GADRET, Amandine, 04100 Manosque (FR)
(74) Mandataire: Cabinet Becker et Associés
(86) Numéro de dépôt international: PCT/FR2018/051229
(87) Numéro de publication internationale: WO 2018/220314

(56) Documents cités:
- WO-A1-2016/185113
- DE-A1-102015 223 837
- US-A- 4 534 962
- US-A- 5 614 179

## Description

### OBJET DE L'INVENTION

La présente invention concerne une composition cosmétique aqueuse à base de sel de bicarbonate, stabilisée par un couple de gélifiants particuliers, ainsi que son utilisation cosmétique en tant que déodorant. Elle concerne également un déodorant à bille renfermant cette composition.

### ARRIERE-PLAN DE L'INVENTION

Les déodorants et antiperspirants sont devenus des produits d'hygiène incontournables. Si les premiers agissent sur les odeurs de transpiration soit en les camouflant, soit en ciblant les bactéries se nourrissant de la sueur apocrine, soit encore en absorbant la transpiration, les seconds régulent la quantité de sueur émise. L'innocuité des sels d'aluminium utilisés comme agents anti-transpirants a été remise en cause ces dernières années, ce qui a conduit les consommateurs à se tourner vers des produits renfermant des agents déodorants naturels tels que le talc et/ou certaines plantes sous forme séchée ou sous forme d'huiles essentielles. L'efficacité de ces produits n'est toutefois pas toujours optimale.

Le bicarbonate de sodium constitue un actif déodorant naturel reconnu. Aux doses considérées comme efficaces, sa formulation est en revanche compliquée par son incompatibilité avec de nombreuses matières premières. Il a en particulier tendance à se dégrader dans les solutions aqueuses ou hydro-alcooliques, dans lesquelles il est par ailleurs difficile à solubiliser. Outre la sensation désagréable qu'elle provoque sur la peau, la présence de cristaux de bicarbonate de sodium est notamment problématique lorsque ce composé est introduit dans des compositions distribuées dans des déodorants à bille, en raison de la propension de ces cristaux propension à bloquer la bille.

Pour cette raison, les déodorants à bille renferment une quantité limitée (inférieure à 3% en poids) de bicarbonate de sodium, ou bien il est recommandé de les agiter avant emploi.

Une solution pour remédier à ce problème est proposée dans le document US-4,534,962. Elle consiste à formuler les sels de bicarbonate en milieu hydro-alcoolique en présence d'un agent de suspension particulier, l'hydroxyéthylcellulose, et éventuellement de polyols. Il est indiqué dans ce document que les autres dérivés de cellulose, et en particulier la carboxyméthyl cellulose, ne permettent pas d'éviter la sédimentation du bicarbonate de sodium. Il est par ailleurs crucial que la composition renferme de grandes quantités d'alcool (au moins 50%). La solution proposée n'est donc pas satisfaisante, en raison des irritations que la composition est susceptible de provoquer à l'application, en particulier sur des peaux récemment épilées.

Une solution alternative est présentée dans le document US-5,614,179, qui consiste à enrober des cristallites de bicarbonate d'un mélange de parfum et de polymère, généralement par atomisation. Le polymère peut être choisi parmi une diversité de matériaux d'origine naturelle ou synthétique, dont les gommes végétales, la gomme de xanthane et les dérivés de cellulose. Ce procédé a l'inconvénient d'être complexe et affecte donc négativement le coût de fabrication des déodorants qui le mettent en œuvre. En outre, les cristallites enrobées sont destinées à être incorporées dans des compositions anhydres renfermant des taux élevés d'huiles volatiles et non dans des gels aqueux ou des émulsions à phase aqueuse continue, qui sont recherchés pour l'effet de fraîcheur qu'ils apportent.

### RESUME DE L'INVENTION

Après de nombreuses recherches, la Demanderesse a mis au point une association de gélifiants permettant de formuler des sels de bicarbonate dans des compositions à phase aqueuse continue, en évitant les problèmes de sédimentation et/ou de cristallisation de ces sels. En outre, les sels de bicarbonate se trouvent en partie solubilisés dans l'eau et en partie en suspension dans la composition, sans affecter négativement la viscosité de la composition.

Ces compositions peuvent en particulier être utilisées dans la fabrication de déodorants à bille (ou « roll-on »), qu'il n'est pas nécessaire d'agiter avant utilisation. Ces compositions sont donc faciles d'application, efficaces contre les odeurs de transpiration et capables de déposer sur la peau un film de texture lisse et fluide.

L'invention a ainsi pour objet une composition cosmétique renfermant :
(a) de 3 à 10% en poids d'au moins un sel de bicarbonate,
(b) de la gomme de xanthane,
(c) au moins un homopolymère de glucose éventuellement carboxyméthylé, et
(d) de 40 à 95% en poids d'eau,
les pourcentages ci-dessus étant exprimés par rapport au poids total de la composition.

Elle a également pour objet l'utilisation cosmétique de cette composition pour traiter les odeurs corporelles humaines, en particulier les odeurs axillaires.

Elle concerne enfin un déodorant à bille renfermant cette composition.

### DESCRIPTION DETAILLEE

La composition selon l'invention est une composition renfermant de 40 à 95% en poids d'eau, de préférence de 50 à 85% en poids d'eau et plus préférentiellement de 60 à 80% en poids d'eau. Il peut s'agir soit d'un gel aqueux, soit d'une émulsion huile-dans-eau. Dans tous les cas, il s'agit d'une composition à phase aqueuse continue.

Cette composition comprend un sel de bicarbonate, utilisé comme agent déodorant. Celui-ci peut représenter de 3 à 10 % en poids, et de préférence de 4 à 6% en poids, par rapport au poids total de la composition. Comme sels de bicarbonate, on peut citer les sels de sodium, de potassium, de magnésium et d'ammonium, le sel de sodium étant préféré pour une utilisation dans cette invention. Selon un mode de réalisation, la composition ne renferme pas de sels d'aluminium. En revanche, elle peut toutefois contenir au moins un actif déodorant additionnel choisi parmi : les agents bactériostatiques ou bactéricides, tels que la chlorhexidine et ses sels ; le triclosan ; le triclocarban ; le farnesol ; les huiles essentielles d'origine végétale, choisies par exemple parmi les huiles essentielles d'origan, de palmarosa, de menthe poivrée, de lavande, de citron et d'arbre à thé ; les extraits végétaux tels que les extraits de graines de pamplemousse ; les sels de zinc tels que le gluconate, le pidolate et le ricinoléate de zinc ; et leurs mélanges.

Comme indiqué précédemment, il a été observé que la composition selon l'invention était stable en présence de bicarbonate, en ce sens qu'on n'observe pas de cristallisation et/ou de sédimentation du bicarbonate et que la texture de la composition reste fluide, sans variation notable de sa viscosité au cours du temps. Cet effet est obtenu par l'ajout d'une combinaison de gélifiants particuliers, à savoir une gomme de xanthane et un homopolymère de glucose éventuellement carboxyméthylé, désigné ci-après pour plus de simplicité par « homopolymère de glucose ».

L'homopolymère de glucose peut être choisi parmi la cellulose, telle que la cellulose microcristalline, qui est la fraction cristalline colloïdale isolée de fibres de cellulose ; la gomme de cellulose ou carboxyméthyl cellulose ; les amidons carboxyméthylés ; et leurs mélanges. Il se différencie des copolymères de glucose tels que la gomme de gellane. Un exemple de mélange de cellulose microcristalline et de carboxyméthyl cellulose, renfermant une fraction majoritaire en poids de cellulose microcristalline, est le mélange disponible auprès de la société FMC BioPolymères sous la dénomination commerciale Avicel® PC 611. Les amidons sont de préférence choisis parmi l'amidon de maïs, de riz, de tapioca ou de blé. Un exemple d'amidon carboxyméthylé est celui commercialisé par la société J. RETTENMAIER sous la dénomination commerciale Vivapharm® CS 152HV.

Il est entendu que l'homopolymère de glucose précité est présent en tant que constituant distinct du sel de bicarbonate, dans la composition selon l'invention, et qu'il ne constitue pas un enrobage de ce dernier. D'une manière générale, on préfère que les particules de bicarbonate présentes dans la composition selon l'invention, en plus du bicarbonate solubilisé dans l'eau, ne soient pas enrobées.

L'homopolymère de glucose peut représenter de 0,3 à 3% en poids et de préférence de 0,3 à 1% en poids dans le cas de l'amidon carboxyméthylé et de 1,2 à 2% en poids dans le cas du mélange de cellulose microcristalline et de gomme de cellulose, par rapport au poids total de la composition.

De son côté, la gomme de xanthane peut représenter de 0,1 à 2% en poids et de préférence de 0,2 à 0,4% en poids, par rapport au poids total de la composition.

Dans un mode de réalisation, la composition selon l'invention peut en outre contenir au moins un gélifiant de phase aqueuse additionnel, en plus de la gomme de xanthane et de l'homopolymère de glucose. Ce gélifiant additionnel peut en particulier être choisi parmi les polymères synthétiques, plus particulièrement parmi les polymères acryliques et préférentiellement parmi les copolymères d'acide (méth)acrylique et de (méth)acrylate d'alkyle en C₁₀₋₃₀. Un exemple d'un tel copolymère amphiphile est celui commercialisé par la société LUBRIZOL sous la dénominations commerciales Carbopol Ultrez® 20 Polymer,

La composition selon l'invention ne renferme avantageusement pas de tensioactif au sens des composés listés dans le Dictionnaire McCUTCHEON « Emulsifiers & Detergents », Edition 2017.

On préfère en outre qu'elle ne contienne pas d'huile et constitue ainsi un gel aqueux. En variante toutefois, la composition selon l'invention peut contenir au moins une huile. Dans ce cas, elle peut ou non inclure un tensioactif.

Dans la présente description, on entend par "huile" un composé liquide à température ambiante (25°C) et pression atmosphérique (10⁵ Pa) qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau. Les huiles peuvent être volatiles ou non volatiles. Par "huile non volatile", on entend dans cette description une huile restant sur la peau à 25°C et pression atmosphérique pendant au moins une heure, en l'absence de frottement, et/ou ayant une pression de vapeur inférieure à 0,001 mm Hg dans ces conditions. Les huiles incluses dans la composition selon l'invention peuvent ou non être volatiles ; elles sont avantageusement non volatiles. En variante, il peut s'agit d'un mélange d'huiles non volatiles (majoritaires en poids) et volatiles (minoritaires en poids). Des exemples d'huiles volatiles sont notamment les alcanes linéaires en C11 à C14 et les isoparaffines. En outre, on préfère que les huiles non volatiles soient choisies parmi les huiles hydrocarbonées, c'est-à-dire qu'elles renferment exclusivement des atomes de carbone, d'hydrogène et éventuellement d'oxygène.

Des exemples d'huiles non volatiles comprennent :
- les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4 ;
- les triglycérides d'acides gras en C6-C12, tels que les triglycérides d'acides caprylique et caprique et la triheptanoïne ;
- les acides gras ramifiés et/ou insaturés en C10-C20 (tels que les acides linoléique, laurique et myristique) ;
- les alcools gras ramifiés et/ou insaturés en C10-C20 (tels que l'octyldodécanol et l'alcool oléylique) ;
- les hydrocarbures tels que le squalane végétal extrait de l'huile d'olive ;
- les carbonates de dialkyle, tels que le dicaprylyl carbonate et le diéthylhexyl carbonate ;
- les dialkyléthers tels que le dicaprylyl éther ; et
- leurs mélanges.

On peut également citer les huiles végétales qui contiennent un ou plusieurs des constituants précités.

Comme esters d'acides et de monoalcools, on peut notamment citer les monoesters tels que le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le caprylate de propylheptyle et leurs mélanges. D'autres esters utilisables sont les diesters d'acides et de monoalcools tels que l'adipate de diisopropyle, l'adipate de diéthylhexyle, le sébaçate de diisopropyle et le sébaçate de diisoamyle.

Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de lavande, de bourrache, de millet, d'orge, de quinoa, de seigle, de carthame, de bancoulier, de passiflore, de rosier muscat ou de camélia.

Outre les huiles ci-dessus, la composition selon l'invention peut en outre contenir au moins un beurre végétal. Par « beurre végétal », on entend un corps gras pâteux à changement d'état liquide / solide réversible, présentant à l'état solide une organisation cristalline anisotrope et comportant à une température de 23°C une fraction liquide et une fraction solide. Des exemples de beurres végétaux sont les beurres de karité, de cacao et de mangue, et leurs mélanges.

Dans une forme d'exécution préférée de l'invention, la composition renferme en outre au moins un polyol tel que la glycérine, le propylène glycol (propane-1,2-diol), le dipropylène glycol, le propane-1,3-diol, le butane-1,2-diol, le butane-1,4-diol, le butane-2,3-diol, le butane-1,3-diol, le pentane-1,5-diol, le pentane-1,2-diol, l'hexane-1,6-diol, l'octane-1,8-diol, le 2-méthylpentane-2,4-diol, le méthylpropanediol, l'isopentyldiol ou un mélange de ceux-ci. Les glycols tels que le propylène glycol sont particulièrement préférés. Le polyol peut représenter de 10 à 50% en poids, de préférence de 20 à 30% en poids, par rapport au poids total de la composition.

On préfère par ailleurs que la composition ne renferme pas de mono-alcool, en particulier pas d'éthanol.

D'autres constituants optionnels pouvant être inclus dans la composition selon l'invention sont les charges pulvérulentes, qui sont adaptées à absorber l'humidité et la sueur et qui se présentent généralement sous forme de microparticules poreuses ou creuses, de préférence poreuses. Ces microparticules sont en principe sensiblement sphériques. Ces charges peuvent notamment être choisies parmi :
- les charges organiques telles que : les poudres de polysaccharides et en particulier d'amidon, tel que l'amidon de maïs, de riz, de tapioca ou de blé ; les poudres de polymères acryliques tels que le poly(méthacrylate de méthyle), de polyamides ou de polyoléfines ; les poudres d'algues séchées telles que *Corallina officinalis* ;
- les charges inorganiques telles que la silice, les argiles, la perlite et le talc ;
- et leurs mélanges.

Ces charges peuvent représenter de 2 à 20% en poids, et de préférence de 2 à 5% en poids, par rapport au poids total de la composition

La composition selon l'invention peut en outre comprendre des additifs choisis notamment parmi des parfums, des anti-oxydants tels que le tocophérol, des colorants, des conservateurs et leurs mélanges.

Le pH de la composition est avantageusement compris entre 7,5 et 9 et peut être ajusté par ajout d'un acide organique tel que l'acide citrique.

La composition selon l'invention se présente sous forme de gel ou de crème dont la viscosité est avantageusement comprise entre 1.500 et 4.000 centipoises, telle que mesurée à l'aide d'un rhéomètre Brookfield® équipé d'un mobile LV3 (réglé à la vitesse 12) à 20°C..

Elle peut être utilisée comme produit déodorant, en particulier dans un déodorant à bille ou « roll-on ».

### EXEMPLES

L'invention sera mieux comprise à la lumière des exemples suivants.

### Exemple 1 : Gel aqueux

On a préparé un gel par mélange des ingrédients suivants, dans les proportions pondérales indiquées ci-dessous.

| | |
|---|---|
| SODIUM BICARBONATE | 5 % |
| MICROCRISTALLINE CELLULOSE & CELLULOSE GUM | 1-2 % |
| XANTHAN GUM | 0,2-1 % |
| PROPYLENE GLYCOL | 20 % |
| CITRIC ACID | qs |
| Gélifiant acrylique | 0,1-0,5 % |
| Parfum(s) | qs |
| Conservateur(s) | qs |
| Eau | qsp 100 % |

Ce gel a été préparé de la manière suivante : la combinaison de gélifiants selon l'invention et le propylène glycol ont été successivement mélangés au gélifiant acrylique préalablement dispersé dans l'eau à 80°C, puis la température a été abaissée à 50°C et le bicarbonate de sodium a été introduit, suivi des conservateurs. La température a encore été réduite à 38°C avant d'ajouter les parfums.

Cette composition a été coulée à température ambiante dans un flacon pourvu d'une bille de distribution.

Analyse sensorielle : cette composition a été testée pendant 1 semaine par un panel de 20 volontaires, dont 70% ont apprécié son efficacité et 85% ont jugé sa texture agréable.

### Exemple 2 : Emulsion

On a préparé une émulsion fluide par mélange des ingrédients suivants, dans les proportions pondérales indiquées ci-dessous.

| | |
|---|---|
| SODIUM BICARBONATE | 5 % |
| MICROCRISTALLINE CELLULOSE & CELLULOSE GUM | 1-2 % |
| XANTHAN GUM | 0,2-1 % |
| PROPYLENE GLYCOL | 20 % |
| CAPRYLIC/CAPRIC TRIGLYCERIDE | 5 % |
| Gélifiant acrylique | 0,1-0,5 % |
| CITRIC ACID | qs |
| Conservateur(s) | qs |
| Eau | qsp 100 % |

### Exemple 3 : Test de stabilité

On a prélevé quatre échantillons A à D des compositions des Exemples 1 et 2, qui ont été répartis dans quatre piluliers stockés respectivement à 4°C, 25°C, 40°C et 50°C. La stabilité des échantillons 1A à 1C et 2A à 2C est évaluée chaque semaine pendant 1 mois puis tous les 15 jours pendant 3 mois et celle des échantillons 1D et 2D est évaluée chaque semaine pendant 1 mois.

On a observé l'aspect des échantillons testés. Les résultats sont présentés dans le Tableau 1 ci-dessous :

**Tableau 1**

| Produit testé | Après 3 mois à 4°C | Après 3 mois à 25°C | Après 3 mois à 40°C | Après 1 mois à 50°C |
|---|---|---|---|---|
| Exemple 1 | stable | stable | stable | stable |
| Exemple 2 | stable | stable | stable | stable |

Il ressort de ce test que les produits selon l'invention sont stables à 40°C pendant 3 mois et à 50°C pendant 1 mois, c'est-à-dire dans des conditions de vieillissement accéléré qui sont considérées comme représentatives du comportement du produit stocké pendant trois ans dans des conditions normales de conservation.

### Exemple 4 : Exemple comparatif

On a évalué la stabilité de plusieurs compositions similaires à celle de l'Exemple 1, en substituant à la gomme de xanthane :
- soit de la gomme de gellane (Ex. A), qui est un hétéropolyoside formé de monomères de D-glucose, L-rhamnose et acide D-glucuronique (Kelcogel® CG-HA de CP KELCO),
- soit un carbomer (Ex. B), c'est-à-dire un homopolymère d'acide acrylique (Carbopol Ultrez® 30 de LUBRIZOL),
- soit un amidon carboxyméthylé (Ex. C) (Vivapharm® CS 152HV de J. RETTENMAIER).

Des formules analogues ont été préparées, qui soit ne contenaient pas de propylène glycol (respectivement Ex. 1-1, A-1, B-1 et C-1), soit renfermaient 30% de propylène glycol au lieu de 20% (respectivement Ex. 1-2 et C-2), et elles ont été testées dans les mêmes conditions.

Ces compositions ont subi le même protocole de vieillissement que celui décrit ci-dessus et ont ensuite été évaluées à l'œil nu et éventuellement observées au microscope pour vérifier la présence ou l'absence de sédimentation du bicarbonate.

Les résultats de ces essais sont rassemblés dans le Tableau 2 ci-dessous.

**Tableau 2**

| Produit testé | Observations |
|---|---|
| Ex. 1 | Stable |
| Ex. 1-1 | Stable |
| Ex. 1-2 | Stable |

| Ex. A | aspect flan, déphase à 50°C |
|---|---|
| Ex. A-1 | aspect flan |
| Ex. B | aspect flan avec relargage |
| Ex. B-1 | aspect flan avec relargage |
| Ex. C | Stable |
| Ex. C-1 | Stable |
| Ex. C-2 | Stable |

Dans le cas des produits comparatifs, on observait un phénomène de synérèse, se traduisant par la présence d'une phase gel recouverte d'eau.

## Revendications

1. Composition cosmétique renfermant :
(a) de 3 à 10% en poids d'au moins un sel de bicarbonate,
(b) de la gomme de xanthane,
(c) au moins un homopolymère de glucose éventuellement carboxyméthylé, et
(d) de 40 à 95% en poids d'eau,
les pourcentages ci-dessus étant exprimés par rapport au poids total de la composition.

2. Composition selon la revendication 1, **caractérisée en ce que** le sel de bicarbonate représente de 4 à 6% en poids, par rapport au poids total de la composition.

3. Composition selon l'une quelconque des revendications 1 ou 2, **caractérisée en ce qu'**elle renferme en outre au moins un polyol tel que la glycérine, le propylène glycol (propane-1,2-diol), le dipropylène glycol, le propane-1,3-diol, le butane-1,2-diol, le butane-1,4-diol, le butane-2,3-diol, le butane-1,3-diol, le pentane-1,5-diol, le pentane-1,2-diol, l'hexane-1,6-diol, l'octane-1,8-diol, le 2-méthylpentane-2,4-diol, le méthylpropanediol, l'isopentyldiol ou un mélange de ceux-ci, de préférence le propylène glycol.

4. Composition selon la revendication 3, **caractérisée en ce que** le polyol représente de 10 à 50% en poids, de préférence de 20 à 30% en poids, par rapport au poids total de la composition.

5. Composition selon l'une quelconque des revendications 1 à 4, **caractérisée en ce que** l'homopolymère de glucose éventuellement carboxyméthylé est choisi parmi la cellulose, la carboxyméthyl cellulose, l'amidon carboxyméthylé et leurs mélanges.

6. Composition selon la revendication 5, **caractérisée en ce qu'**elle renferme un mélange de cellulose et de carboxyméthyl cellulose.

7. Composition selon la revendication 5, **caractérisée en ce qu'**elle renferme un amidon carboxyméthylé.

8. Composition selon l'une quelconque des revendications 1 à 7, **caractérisée en ce qu'**elle est exempte d'huile et/ou d'éthanol.

9. Composition selon l'une quelconque des revendications 1 à 8, **caractérisée en ce qu'**elle renferme de 50 à 85% et de préférence de 60 à 80% en poids d'eau.

10. Déodorant à bille renfermant une composition selon l'une quelconque des revendications 1 à 9.

11. Utilisation cosmétique de la composition selon l'une quelconque des revendications 1 à 9 pour traiter les odeurs corporelles humaines, en particulier les odeurs axillaires.

## Patentansprüche

1. Kosmetische Zusammensetzung umfassend:
(a) 3 bis 10 Gew.-% wenigstens eines Bicarbonat-Salzes,
(b) Xanthangummi,
(c) wenigstens ein gegebenenfalls carboxymethyliertes Glucose-Homopolymer und
(d) 40 bis 95 Gew.-% Wasser,
wobei die obigen Prozentangaben bezogen auf das Gesamtgewicht der Zusammensetzung ausgedrückt sind.

2. Zusammensetzung gemäß Anspruch 1, **dadurch gekennzeichnet, dass** das Bicarbonat-Salz 4 bis 6 Gew.-% bezogen auf das Gesamtgewicht der Zusammensetzung darstellt.

3. Zusammensetzung gemäß einem der Ansprüche 1 oder 2, **dadurch gekennzeichnet, dass** sie außerdem wenigstens ein Polyol wie Glycerin, Propylenglycol, (Propan-1,2-diol), Dipropylenglycol, Propan-1,3-diol, Butan-1,2-diol, Butan-1,4-diol, Butan-2,3-diol, Butan-1,3-diol, Pentan-1,5-diol, Pentan-1,2-diol, Hexan-1,6-diol, Octan-1,8-diol, 2-Methylpentan-2,4-diol, Methylpropandiol, Isopentyldiol oder ein Gemisch davon, vorzugsweise Propylenglycol umfasst.

4. Zusammensetzung gemäß Anspruch 3, **dadurch gekennzeichnet, dass** das Polyol 10 bis 50 Gew.-%, vorzugsweise 20 bis 30 Gew.-% bezogen auf das Gesamtgewicht der Zusammensetzung darstellt.

5. Zusammensetzung gemäß einem der Ansprüche 1 bis 4, **dadurch gekennzeichnet, dass** das gegebenenfalls carboxymethylierte Glucose-Homopolymer aus Cellulose, Carboxymethylcellulose, carboxymethylierter Stärke und ihren Gemischen ausgewählt ist.

6. Zusammensetzung gemäß Anspruch 5, **dadurch gekennzeichnet, dass** sie eine Mischung aus Cellulose und Carboxymethylcellulose umfasst.

7. Zusammensetzung gemäß Anspruch 5, **dadurch gekennzeichnet, dass** sie eine carboxymethylierte Stärke umfasst.

8. Zusammensetzung gemäß einem der Ansprüche 1 bis 7, **dadurch gekennzeichnet, dass** sie frei von Öl und/oder Ethanol ist.

9. Zusammensetzung gemäß einem der Ansprüche 1 bis 8, **dadurch gekennzeichnet, dass** sie 50 bis 85 Gew.-% und vorzugsweise 60 bis 80 Gew.-% Wasser umfasst.

10. Deoroller umfassend eine Zusammensetzung gemäß einem der Ansprüche 1 bis 9.

11. Kosmetische Verwendung der Zusammensetzung gemäß einem der Ansprüche 1 bis 9 zur Behandlung von menschlichen Körpergerüchen, insbesondere Achselgeruch.

## Claims

1. A cosmetic composition containing:
(a) from 3 to 10% by weight of at least one bicarbonate salt,
(b) xanthan gum,
(c) at least one optionally carboxymethylated glucose homopolymer, and
(d) from 40 to 95% by weight of water,
the above percentages being expressed relative to the total weight of the composition.

2. The composition as claimed in claim 1, **characterized in that** the bicarbonate salt represents from 4 to 6% by weight, relative to the total weight of the composition.

3. The composition as claimed in either one of claims 1 and 2, **characterized in that** it also contains at least one polyol, such as glycerol, propylene glycol (propane-1,2-diol), dipropylene glycol, propane-1,3-diol, butane-1,2-diol, butane-1,4-diol, butane-2,3-diol, butane-1,3-diol, pentane-1,5-diol, pentane-1,2-diol, hexane-1,6-diol, octane-1,8-diol, 2-methylpentane-2,4-diol, methylpropanediol, isopentyldiol or a mixture thereof, preferably propylene glycol.

4. The composition as claimed in claim 3, **characterized in that** the polyol represents from 10 to 50% by weight, preferably from 20 to 30% by weight, relative to the total weight of the composition.

5. The composition as claimed in any one of claims 1 to 4, **characterized in that** the optionally carboxymethylated glucose homopolymer is chosen from cellulose, carboxymethylcellulose, carboxymethylated starch, and mixtures thereof.

6. The composition as claimed in claim 5, **characterized in that** it contains a mixture of cellulose and carboxymethylcellulose.

7. The composition as claimed in claim 5, **characterized in that** it contains a carboxymethylated starch.

8. The composition as claimed in any one of claims 1 to 7, **characterized in that** it is free of oil and/or of ethanol.

9. The composition as claimed in any one of claims 1 to 8, **characterized in that** it contains from 50 to 85% and preferably from 60 to 80% by weight of water.

10. A roll-on deodorant containing a composition as claimed in any one of claims 1 to 9.

11. The cosmetic use of the composition as claimed in any one of claims 1 to 9 for treating human body odor, in particular armpit odor.
